Europäisches Patentamt

European Patent Office

Office européen des brevets

(19)

(11) **EP 0 806 910 B1**

(12) **FASCICULE DE BREVET EUROPEEN**

(45) Date de publication et mention
de la délivrance du brevet:
**09.04.2003 Bulletin 2003/15**

(51) Int Cl.[7]: **A61B 8/12**

(86) Numéro de dépôt international:
**PCT/FR96/00155**

(21) Numéro de dépôt: **96902308.4**

(22) Date de dépôt: **30.01.1996**

(87) Numéro de publication internationale:
**WO 96/023444 (08.08.1996 Gazette 1996/36)**

(54) **CATHETER D'IMAGERIE ECHOGRAPHIQUE ENDOCAVITAIRE**

KATHETER ZUR ABBILDUNG VON KÖRPERHÖHLEN MITTELS ULTRASCHALLECHO

INTRACAVITARY ECHOGRAPHIC IMAGING CATHETER

(84) Etats contractants désignés:
**DE ES FR GB IT NL**

(30) Priorité: **01.02.1995 FR 9501173**

(43) Date de publication de la demande:
**19.11.1997 Bulletin 1997/47**

(73) Titulaire: **CENTRE NATIONAL DE
LA RECHERCHE SCIENTIFIQUE
75016 Paris Cédex (FR)**

(72) Inventeurs:
• **BERGER, Geneviève
F-92340 Bourg-La-Reine (FR)**
• **BOVO, Nicolas
F-95400 Arnouville-les-Gonesse (FR)**
• **DUFOUR, Isabelle
F-92220 Bagneux (FR)**
• **GRANDCHAMP, Jean-Paul
F-91190 Gif-sur-Yvette (FR)**
• **LAUGIER, Pascal
F-75017 Paris (FR)**
• **SOL, Charles
F-91320 Wissous (FR)**
• **ALLANO, Sylvain
F-91310 Montlhery (FR)**

(74) Mandataire: **Armengaud Ainé, Alain et al
Cabinet ARMENGAUD AINE
3 Avenue Bugeaud
75116 Paris (FR)**

(56) Documents cités:
**WO-A-91/02488        WO-A-93/05712
NL-A- 8 700 632        US-A- 3 948 350
US-A- 4 479 388        US-A- 5 168 878
US-A- 5 313 950**

• **INTERNATIONAL ELECTRON DEVICES
MEETING TECHNICAL DIGEST, Décembre 1988,
US, pages 666-669, XP000040502 FAN ET AL.:
"IC-processed electrostatic micro-motors"**

## Description

La présente invention concerne un cathéter d'imagerie échographique endocavitaire.

On sait que le dépistage et l'évaluation de la sévérité de certaines pathologies sont basées sur l'analyse d'images échographiques. Ainsi, on a été amené à développer l'imagerie ultrasonore intravasculaire pour la caractérisation des lésions athéromateuses chez l'homme. En effet, La résolution que l'on peut obtenir avec les ultrasons dépend de la fréquence utilisée, plus cette fréquence est élevée, meilleure est la résolution. Cependant, l'atténuation que subit le faisceau d'ultrasons croît avec la fréquence, et la profondeur de pénétration du faisceau diminue considérablement. Une image de très bonne résolution à haute fréquence ne peut donc être obtenue que par voie endovasculaire.

Le traitement des lésions dues à la présence de plaques d'athéromes dans les artères coronaires fait appel à deux techniques invasives distinctes : la chirurgie et l'intervention par cathéter (angioplastie par ballonnet ou laser). L'utilisation d'outils cathétérisés représente une alternative confortable. Toutefois, la chirurgie s'avère nécessaire lorsque les lésions sont graves, c'est-à-dire, dans un état avancé. Dans ce cas, les interventions chirurgicales consistent à remplacer la portion d'artère coronaire sténosée par une partie d'une autre artère, soit par prélèvement et greffe, ce qui réalise un pontage de la zone, soit par utilisation d'une autre artère pour irriguer le coeur.

Les cathéters sont utilisés pour les interventions mettant en oeuvre la technique d'angioplastie par ballonnet. Selon cette technique, on introduit un cathéter d'environ 1,5 mètre de long et 1 mm de diamètre, par voie percutanée dans l'artère fémorale et on accède à l'artère coronaire en suivant un cheminement dans le réseau artériel. Le guidage de la sonde s'effectue à l'aide d'un fil guide solidaire ou non du cathéter et dont l'extrémité est recourbée afin d'éviter les perforations lors des manipulations nécessaires à la progression et à l'orientation du cathéter. Ce cheminement est suivi par une technique d'imagerie externe, ce qui favorise et sécurise la mise en place. La thérapie peut également être basée sur l'abrasion de la plaque d'athérome par laser ou mini scalpel, dont le guidage est réalisé selon le même principe que pour les cathéters d'angioplastie.

Les critères du choix du recours à l'une ou l'autre des méthodes mentionnées ci-dessus sont empiriques. Ils tiennent compte de connaissances à priori sur la structure de la plaque et sur son importance en épaisseur et en longueur. Malgré la préférence pour l'angioplastie, en raison de sa plus grande simplicité, les deux méthodes comportent cependant des risques importants.

La maniabilité du cathéter, la simplification des procédures interventionnelles, l'augmentation de la cellule de résolution et la connaissance de la position de l'extrémité distale du cathéter sont les points clef du développement de cette technique de contrôle thérapeutique.

L'échographie intra-coronarienne se caractérise par les faibles dimensions des éléments à explorer et des chemins pour y accéder. Cela se traduit par un cahier des charges tout à fait spécifique pour la conception des cathéters, des éléments piezo-électriques, des éléments permettant le balayage et des outils de traitement des sténoses.

Par ailleurs, l'échographie quantitative a pour but l'exploitation complète du signal échographique et l'extraction des paramètres quantitatifs d'aide au diagnostic apportant une information supplémentaire par rapport à l'image échographique classique. Ainsi, les méthodes de caractérisation tissulaires par ultrasons permettent d'estimer des paramètres quantitatifs à partir du signal échographique ou de l'image échographique. Ces méthodes exigent également des caractéristiques spécifiques en ce qui concerne les systèmes permettant d'obtenir les signaux ou images échographiques.

Ainsi, le câble du cathéter dont la longueur totale est d'environ 1,50 mètre présente une partie proximale suffisamment rigide pour permettre le guidage et une partie distale plus flexible présentant un diamètre hors tout de 1 à 2 mm et une longueur de 2 à 15 cm. L'extrémité distale est constituée d'une capsule transparente aux ultrasons et qui contient le système échographique immergé dans un liquide de couplage. L'ensemble doit être compatible avec les milieux biologiques et l'on utilise généralement, pour sa réalisation, des polymères tels que silicone, chlorure de polyvinyl, polyéthylène, polyuréthannes, polyesters, polytétrafluoréthylène.

La fréquence centrale des transducteurs large bande utilisés varie de 20 à 40 Mhz et leur tension d'alimentation est comprise entre 100 et 300 V.

Les sondes ultrasonores cathétérisées utilisées pour l'échographie intra-coronarienne peuvent être classifiées selon deux axes : selon le mode de balayage permettant de construire l'image et, d'après les outils thérapeutiques embarqués dans le cathéter.

La première solution pour construire une image selon un plan de coupe consiste à faire tourner l'élément piezo-électrique. La principale caractéristique de cette solution réside dans la nécessité d'avoir des contacts électriques entre une partie mobile, constituée par le transducteur, et une partie fixe constituée par le milieu extérieur. De plus, la distance séparant la source d'ultrasons des tissus à explorer est faible, d'où le risque de se trouver dans la zone de champ proche. Pour résoudre ce dernier problème, on utilise un miroir tournant, plan ou concave, défléchissant les ondes ultrasonores de 45°, ce qui permet, en faisant émettre le transducteur dans l'axe du cathéter, d'allonger la distance entre l'émetteur et les tissus. En conservant cette structure, le transducteur peut être fixé au cathéter, le miroir étant le seul élément embarqué.

Cette dernière solution est celle qui est la plus couramment utilisée. Cependant, l'ensemble mobile constitué d'un transducteur et d'un miroir est utilisé pour une mise en place du cathéter après l'introduction d'un fil guide indépendant.

Pour illustrer l'état de la technique dans ce domaine, on peut citer notamment

- US-A-4 794 931 qui décrit un cathéter pour échographie endocavitaire dont l'extrémité distale est munie d'un transducteur ultrasonore, ce transducteur pouvant être entraîné en rotation par un moteur extérieur afin d'assurer le balayage du faisceau.

- WO 93/05712 qui décrit également un cathéter pour échographie endocavitaire dont l'extrémité distale comporte un transducteur ultrasonore fixe, un miroir de déflection des ondes ultrasonores orienté à 45°, et un micromoteur du type horlogerie sur l'axe duquel est monté ledit miroir, par assemblage a posteriori.

US-A-5 313950 décrit un cathéter pour échographie endocavitaire selon le préambule de la revendication 1 comportant notamment un transducteur piézo-électrique, un miroir de déflection des ondes ultrasonores orienté à 45° et un moteur dont le rotor entrainant le miroir est monté dans la capsule du cathéter, cependant que le stator est extérieur au patient à examiner.

Les systèmes actuellement connus d'échographie cathétérisée présentent notamment les défauts suivants :

- la longueur du cathéter et le passage de celui-ci dans des cavités à géométrie coudée engendrant un couple de torsion variable, il n'y a pas de contrôle de la position angulaire ni de la vitesse de rotation de l'extrémité distale du cathéter, et donc de l'organe d'émission et d'acquisition des ondes ultrasonores,

- les variations de la vitesse de rotation entraînent une distorsion de l'image,

- le cathéter véhicule un arbre mécanique dont la rigidité est antagoniste à la mobilité au travers du réseau artériel sinueux, présentant en outre le risque de dissection de la plaque d'athérome.

- le diamètre élevé de l'extrémité distale du cathéter ne permet qu'une investigation réduite des artères coronaires, se limitant aux zones proximales dont la sténose réduit peu la lumière du vaisseau,

- le manque de maniabilité des dispositifs à balayage mécanique dû à la connexion à un module extérieur de rotation,

- la rigidité de l'extrémité distale des cathéters endo-motorisés, due à la longueur importante du micromoteur et de son arbre (supérieure à 5 mm) ; cette partie n'étant pas flexible et rendant dangereuse la mise en place du cathéter (perforation, décollement de plaques d'athérome),

- l'utilisation d'un micromoteur du type électromagnétique nécessite une compatibilité électromagnétique double avec l'environnement biologique et la capsule distale du cathéter : à la fois, pour le champ électrique et pour le champ magnétique.

Partant de cet état antérieur de la technique, l'invention se propose d'apporter un cathéter d'imagerie échographique endocavitaire de petite taille, ne présentant pas les inconvénients de cette technique antérieure.

Dans ce but, le cathéter selon l'invention est un cathéter d'imagerie échographique endocavitaire comprenant une extrémité proximale située à l'extérieur du patient, une extrémité distale située au voisinage des tissus à explorer et un corps reliant ces deux extrémités , l'extrémité distale comprenant un fil de guide et une capsule transparente aux ultrasons dans laquelle sont logés un transducteur piezo-électrique fixe et un miroir de déflexion de l'onde ultra sonore, entraîné en rotation par un microactionneur dont le rotor et le stator est également logé dans ladite capsule , ledit miroir étant déposé directement, sans arbre de transmission du couple à la surface dudit rotor du microactionneur.

Le microactionneur est un moteur électrostatique fabriqué par des procédés de fabrication collective, sans assemblage, dérivés des procédés utilisés en microélectronique (technologie silicium) et/ou par des procédés spécifiques pour la réalisation de micro-objets tridimensionnels, notamment par électroformage. De préférence, ce moteur électrostatique est à capacité variable et à excitation radiale.

Selon une caractéristique de l'invention, le diamètre extérieur dudit microactionneur est compris entre 0.1 mm et 1 mm, typiquement 0.5 mm et son épaisseur est comprise entre 10 et 100µm.

Selon un mode de réalisation préféré de l'invention, le miroir est constitué d'une plaque à haute impédance acoustique par rapport au liquide intracapsulaire, orientée à environ 45° par rapport à l'axe du transducteur, ladite plaque étant située au sommet d'un cylindre coupé à environ 45° et tournant dans l'axe de la sonde. Selon un exemple de réalisation, cette plaque peut être métallique.

Selon un mode de réalisation préféré permettant d'éliminer le balourd qui résulte du fait que le centre de gravité du cylindre au sommet duquel est placée ladite plaque, n'est pas situé sur l'axe de rotation, ledit miroir est réalisé sous la forme d'un disque posé sur un pied relié directement au rotor du micro-actionneur. Ce disque qui peut être de forme concave ou plane comporte une couche réfléchissante fine, par exemple métallique déposée sur un milieu absorbant les ultrasons.

D'autres caractéristiques et avantages de la présente invention ressortiront de la description faite ci-après en référence aux dessins annexés sur lesquels :

- la figure 1 est une vue schématique en élévation d'un exemple de réalisation d'un cathéter muni d'une sonde micromotorisée selon la présente invention ;

- la figure 2 est une vue en élévation d'un détail de la sonde, représenté à plus grande échelle, ce détail étant constitué par le système miroir-rotor du micromoteur ;

- la figure 3 est une représentation schématique en perspective d'un exemple de réalisation d'un miroir pouvant être mis en oeuvre dans ladite sonde cathétérisée ;

- les figures 4 et 5 sont des vues schématiques, respectivement en perspective et en coupe d'un autre exemple de réalisation dudit miroir ;

- la figure 6 est une vue en plan d'un exemple de réalisation du microsystème: moteur-miroir ;

- la figure 7 est une vue similaire à la figure 6 indiquant les définitions d'angles rotoriques et statoriques du micro-moteur, et;

- la figure 8 illustre en perspective une structure de micro-moteur en cuivre, réalisée par électroformage pouvant être utilisé selon l'invention.

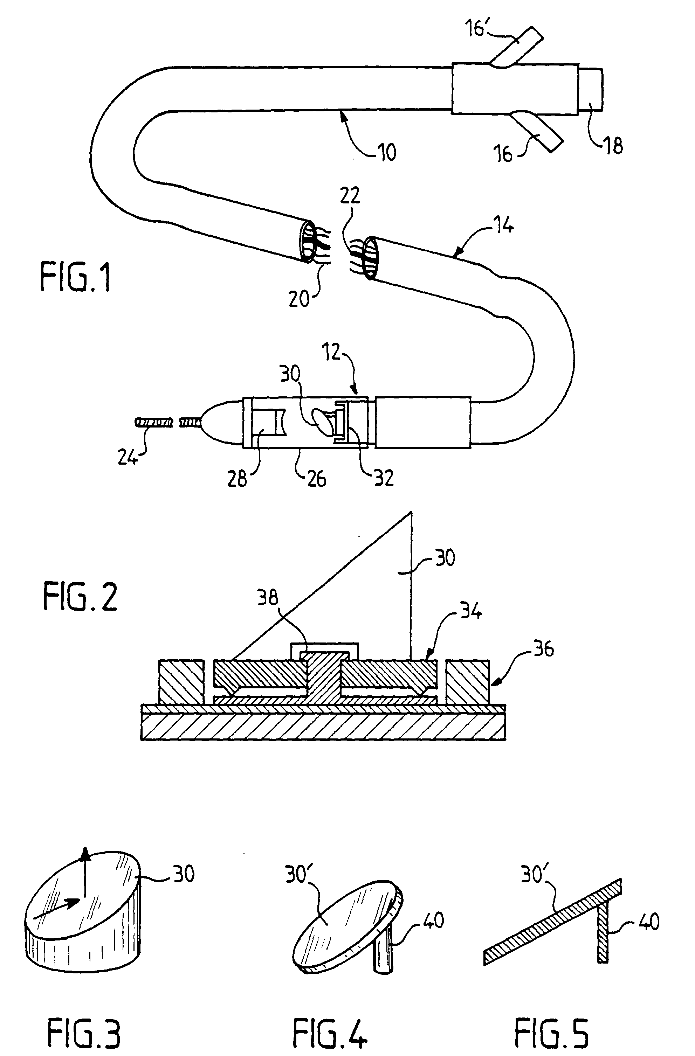

Ainsi qu'on le voit sur la figure 1, le cathéter d'imagerie échographique endocavitaire selon l'invention, comprend une extrémité proximale 10 située à l'extérieur du patient, une extrémité distale 12 située au voisinage des tissus à explorer et un corps 14 reliant ces deux extrémités.

L'extrémité proximale 10 est munie des connections électriques du moyen entraînant en rotation le miroir placé dans la capsule à l'extrémité distale 12, des connections électriques du transducteurs piezo-électrique, également placé dans cette extrémité distale, et les interfaces de liaison entre l'outil thérapeutique et son dispositif de contrôle extérieur (laser, angioplastie par ballonnet ou scalpel de dissection). Sur la figure 1, on a schématisé les conduits 16, 16' et 18 qui sont prévus pour les passages de ces différents cables.

Le corps 14 du cathéter est l'organe de liaison entre la partie active (décrite ci-après) située à l'extrémité distale 12 et le système de commande, d'acquisition et de contrôle situé à l'extérieur du patient. Ce corps, contient les cables électriques 20 d'alimentation du moyen d'entraînement du miroir (décrit ci-après), et du transducteur piezo-électrique, les supports véhiculant les signaux de commande de l'outil thérapeutique, les passages de fluides 22, etc...

L'extrémité distale 12 comprend essentiellement le fil guide 24 et la capsule 26 transparente aux ultrasons dans laquelle est positionné le dispositif d'acquisition échographique spatiale de plans ou de coupes. En arrière de cette capsule, se trouve l'outil de traitement thérapeutique. Le dispositif d'acquisition échographique est constitué du transducteur piezo-électrique fixe, plan ou focalisé 28, et d'un miroir 30 de déflection des ondes ultrasonores, orienté de préférence selon un angle de 45°, entraîné en rotation.

De manière connue, la construction de l'image est réalisée par le balayage de l'onde ultrasonore, à l'extrémité distale 12 du cathéter. Dans ce but, selon l'invention, le dispositif d'acquisition placé dans la capsule 26 comporte le transducteur piezo-électrique fixe axial 28, plan ou focalisé et le miroir 30 assurant la déflection de l'onde ultrasonore, ce miroir étant entraîné en rotation par une machine tournante. La direction du cathéter et celle du vecteur de l'onde émise par le transducteur 28 sont colinéaires mais de sens opposé. La machine tournante, intégrée dans l'extrémité distale du cathéter est, selon un mode de réalisation préféré de l'invention, constituée par un microactionneur rotatif électrostatique fabriqué à l'aide de procédés de fabrication collective dérivés des procédés utilisés en microélectronique. Pour certaines applications dans lesquelles les problèmes de compatibilité électromagnétique n'interviennent pas, on peut réaliser un microactionneur rotatif électromagnétique également fabriqué à partir de procédés de fabrication collective.

Ainsi qu'on le voit sur la figure 2 le miroir 30 est déposé directement sur la surface du rotor 34 du micromoteur électrostatique 32. Grâce à cette disposition, le miroir 30 n'est pas entraîné par l'arbre du moteur : il n'existe pas de transmission de la rotation à l'arbre. L'axe 38 montré dans le figure 2 est fixe et il ne sert que d'axe de centrage et de verrou étant donné que le moteur 32 doit fonctionner dans n'importe quelle position.

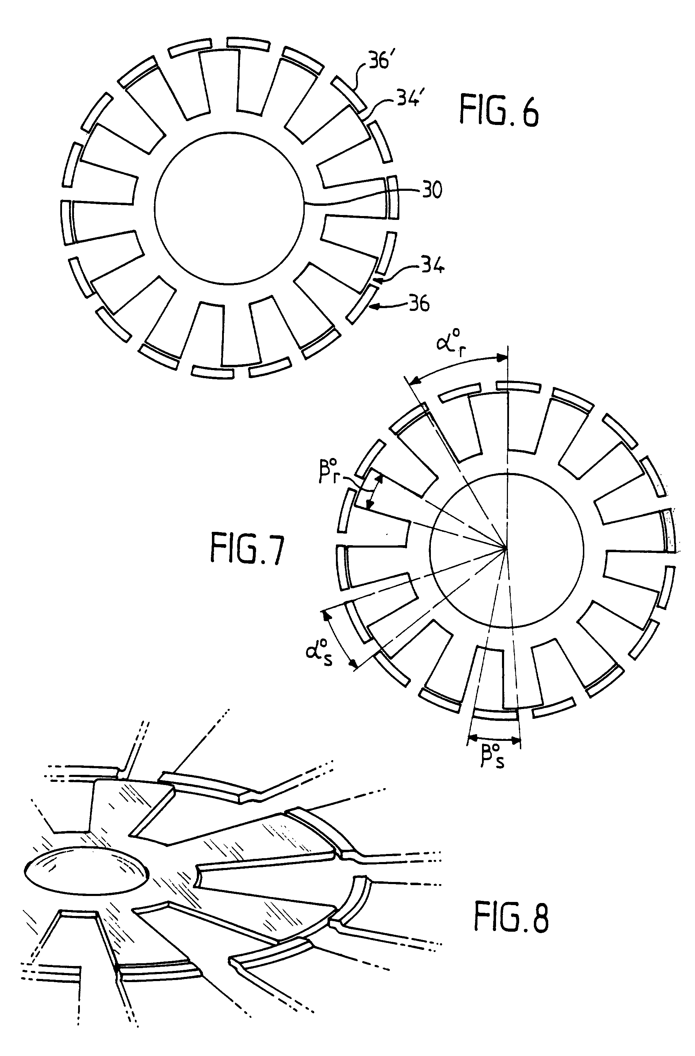

De préférence, le moteur électrostatique 32 est à capacité variable et à excitation radiale. Son diamètre extérieur est compris entre 0.1 et 1 mm, typiquement 0.5 mm et son épaisseur est comprise entre 10 µm et 100µm L'espace entre le stator 36 et le rotor 34 est compris entre 1 et 5 µm. La structure géométrique du moteur (figures 6 et 7) est de préférence de type 3/2 (c'est-à-dire qu'il comporte 3p plots statoriques 36' et 2p dents rotoriques 34', p étant un entier). Le dimensionnement des plots statoriques 36' et des dents rotoriques 34' est tel que : $\beta_r \in [0,3;0,7]$ et $\beta_s \in [0,4;0,9]$ avec typiquement $\beta_r = 0.5$ et $\beta_s = 0.75$.

Si Nr est le nombre de dents rotoriques et Ns, le nombre de plots statoriques, on a (figure 7) :

$$\alpha^{\circ}_r = 2\pi/Nr, \ \alpha^{\circ}_s = 2\pi/Ns$$

$$\beta_r = \beta^{\circ}_r{}^{\circ}/\alpha^{\circ}_r \ \ \beta_s = \beta^{\circ}_s/\alpha^{\circ}_s$$

Les matériaux utilisés pour le stator et le rotor sont du silicium ou des matériaux (purs ou sous forme d'alliages) et des polymères.

Le microsystème contenu dans la capsule 26 est immergé dans un liquide intracapsulaire, assurant le couplage ultrasonore. Ce liquide doit être peu visqueux, non conducteur, peu atténuant avec une permittivité électrique si possible élevée. On peut par exemple utiliser de l'eau désionisée ou des huiles.

Le fait de concevoir un microsystème constitué d'un transducteur fixe 28 et d'un réflecteur ultrasonore rotatif 30 permet d'allonger la distance entre ces deux éléments. En effet, en raison de la proximité de la sonde et des tissus à explorer, il est nécessaire d'augmenter la distance entre cette sonde et ces tissus afin de s'affranchir des problèmes liés à la zone de champ proche. Dans ce cas, les connections du transducteur sont fixes, ce qui élimine toute connection tournante.

Selon un mode de réalisation de l'invention, le miroir 30 peut être réalisé sous la forme (figure 3) d'une plaque, de préférence métallique, à haute impédance acoustique orientée à environ 45° par rapport à l'axe du transducteur 28, cette plaque étant placée au sommet d'un cylindre coupé à environ 45° et entraîné en rotation selon l'axe de la sonde. Cette solution présente toutefois l'inconvénient que le centre de gravité du cylindre n'est pas situé sur l'axe de rotation, ce qui crée un problème de balourd. Ce dernier peut être compensé par exemple, en ajoutant un poids mort afin d'équilibrer la structure. Par ailleurs, la hauteur d'une telle structure cylindrique ne favorise pas sa réalisation technologique conjointe au micromoteur 32. Une construction séparée suivie d'un assemblage augmenterait0 considérablement le coût de l'ensemble du système.

La réduction de la hauteur du cylindre et l'équilibre de cette structure sont les deux contraintes majeures guidant les choix menant à d'autres solutions selon l'invention.

Le balourd enregistré par le décalage existant entre l'axe du moteur 32 et sa parallèle passant par le centre de gravité du cylindre portant le miroir est éliminé par l'alignement de ces deux droites. Cette solution est envisageable dans la mesure où l'augmentation sous-jacente éventuelle du diamètre de l'ensemble permet de respecter le cahier des charges.

Pour réduire le poids du miroir, celui-ci peut être réalisé sous la forme d'un disque miroir 30', à haute impédance acoustique, posé sur un pied 40 relié directement au rotor 34 du micromoteur. Ce mode de réalisation est illustré par les figures 4 et 5.

On peut également remarquer qu'en inclinant les éléments du miroir d'un angle inférieur à 45°, on peut réduire la hauteur de son cylindre porteur. Avec un angle de 30°, la hauteur est pratiquement divisée par 2.

Selon l'invention, le miroir est constitué d'une couche réfléchissante fine, de préférence métallique déposée sur un milieu absorbant les ultrasons. Le miroir peut être de forme plane, cependant, le faisceau réfléchi par ledit miroir peut être focalisé en lui donnant une forme concave, ce qui augmente en outre la résolution du système.

Le miroir est directement fabriqué sur le rotor du micro-moteur par des procédés de fabrication collective dérivés des procédés utilisés en microélectronique (technologie silicium) et/ou par des procédés spécifiques pour la réalisation de micro-objets tridimensionnels, notamment par électroformage. La figure 8 des dessins annexés illustre une structure de micro-moteur en cuivre ainsi obtenue par électro-formage.

La diminution de la taille du miroir, sans perte d'énergie, est un problème qui s'estompe par l'utilisation d'un transducteur focalisé, la taille du faisceau étant plus faible, il est possible de diminuer le diamètre dudit miroir. Les facteurs principaux du dimensionnement sont liés aux possibilités d'exploration des tissus (profondeur de tissu explorée, résolution) limitées par l'atténuation des signaux dans le liquide de couplage, dans le sang et dans les tissus.

Ainsi qu'on le comprend de la description qui précède, l'ensemble constitué du transducteur piezo-électrique 28, du miroir 30 et du microactionneur 32 permettant la rotation du miroir, constitue l'unité d'acquisition ultrasonore du site in vivo du cathéter.

La mise en place de cette unité d'acquisition sur le site est assurée par le fil guide 24 suivi du cathéter. Une fois en place, l'émission et la réception de multiples echos ont lieu au cours de la rotation du miroir 30. La commande du moteur 32, de type pas à pas, permet une information de position précise sur la coupe ainsi réalisée. Les déplacements axiaux du cathéter et l'acquisition de nouveaux plans de coupe permettent une reconstruction précise en trois dimensions ainsi qu'un repérage des sténoses pouvant être traitées par l'unité thérapeutique située à l'extrémité distale du cathéter, et proximale par rapport à l'unité d'acquisition ultrasonore.

Les avantages apportés par l'invention sont notamment les suivants :

- Intégration du système de miroir au microactionneur, avec un couplage direct du miroir au micro-actionneur, sans arbre intermédiaire, réalisant ainsi un microsystème intégré « micromoteur-miroir ».

- Utilisation d'un microactionneur et d'un miroir pouvant être fabriqués ensemble et sans assemblage par un procédé collectif.

- Utilisation d'un miroir de faible poids supporté par un pied dont la zone d'appui ne couvre pas la totalité de la surface utile de ce miroir ;

- Utilisation d'un miroir d'épaisseur faible et constante, pouvant présenter un milieu arrière d'amortissement ;

- Possibilité d'une orientation du miroir inférieure ou égale à 45° ;

- Possibilité de réaliser le traitement des informations directement à l'extrémité distale du cathéter, et sur le même substrat silicium que celui utilisé pour le moteur 32. ce qui permet de réaliser un microsystème « miroir-moteur-traitement, de type ASIC (« Application specific integrated circuit ») ;

- Miniaturisation de l'extrémité distale du cathéter rendant ainsi possible l'accès aux artères coronaires présentant une lumière d'un diamètre inférieur à 1 mm, typiquement 0,6 mm;

- Diminution de la longueur rigide de l'extrémité distale par rapport aux systèmes avec moteur, du type horlogerie, placé en bout de cathéter;

- Diminution de la rigidité du cathéter, et particulièrement de son extrémité distale sans dégradation des caractéristiques tournantes de l'organe de balayage permettant ainsi non seulement d'accéder à des parties de vaisseaux dont la topographie du cheminement est particulièrement compliquée, mais également de diminuer le risque de détacher des fragments de plaques d'athérome dans les artères;

- Maîtrise de la vitesse de rotation du micromoteur et de son maintien à une valeur constante grâce à l'élimination de l'arbre de transmission mécanique entre le moteur et le miroir, responsable du couple de torsion variable.

Tous ces avantages permettent :

- Une diminution des coûts (équipements et examens);

- Un accès précis aux caractéristiques topographiques des tissus observés ; (connaissance de la position absolue du capteur par rapport à la structure anatomique) ;

- La superposition et le recalage de clichés d'après les informations exactes de position tridimensionnelle de chacun d'entre eux, et fusion avec des clichés d'autres modalités d'imagerie ;

- L'extraction de paramètres acoustiques quantitatifs représentatifs de la constitution histologique d'une zone précise. Divers paramètres permettent de caractériser la maladie et d'en évaluer sa gravité : épaisseur des couches, célérité des ultrasons, atténuation, coefficient de rétrodiffusion. Leur représentation sous forme topographique permet de superposer ces différents paramètres aux clichés classiques et aux couches histologiques ;

- La reconstruction tridimensionnelle d'un volume exploré par recalage des différentes coupes d'après l'information précise de la position exacte des unes par rapport aux autres, et ;

**EP 0 806 910 B1**

- La possibilité d'utiliser la place libérée par l'absence d'arbre de transmission mécanique, pour le déplacement dans la troisième dimension (longitudinale) du microactionneur permettant ainsi une acquisition directe d'un volume d'images en trois dimensions.

Le principal domaine d'application de l'invention est celui de l'exploration endovasculaire des artères coronaires. Toutefois, cette application n'est pas limitative, en effet, le dispositif selon l'invention peut être appliqué à l'exploration de tissus non accessibles par voie endocavitaire, notamment à l'exploration percutanée, et en particulier intra-articulaire.

On indiquera maintenant une solution particulière pour un auto-pilotage.

Afin d'obtenir une vitesse de rotation constante, il faut éviter les variations de couple moteur. Pour cela, il faut cesser d'alimenter un plot statorique, au moment où une dent rotorique arrive en conjonction avec celui-ci, puis alimenté le plot statorique suivant. Une telle commande dite auto-pilotée n'est possible que si l'on dispose d'une information sur la position du rotor. Dans la pratique, il n'est pas possible de placer un capteur de position sur le micro-moteur. Il faut donc utiliser les signaux directement issus du micro-moteur pour estimer la position du rotor. La capacité entre le rotor et un plot statorique variant avec la position du rotor, on peut imaginer de connaître la position du rotor en mesurant cette capacité. Dans la pratique, la mesure de position peut donc être faite en mesurant la capacité entre des plots statoriques non alimentés et le rotor. La variation relative de cette capacité est très faible (car les fils et les connexions sont la cause d'une capacité moyenne importante) et on propose donc un montage particulier (amplificateur de gain supérieur à 1 avec une capacité entre l'entrée et la sortie) qui par effet Miller permet de ramener une capacité équivalente négative en parallèle avec la capacité à mesurer. Ainsi, les variations de capacité (dues au mouvement du rotor) sont autour d'une capacité moyenne très faible et la variation relative est donc importante et mesurable (par exemple à l'aide d'un oscillateur dont la fréquence dépend de la capacité suivie d'un démodulateur de fréquence).

Pour ce type de commande auto-pilotée, la fréquence d'alimentation n'étant plus controlée, le réglage de la vitesse s'effectue en agissant sur la valeur de la tension d'alimentation.

Des résultats de simulation ont démontré la validité d'un tel auto-pilotage.

Afin de déterminer la tension d'alimentation nécessaire pour obtenir une vitesse de rotation uniforme de 2 000 tours par minute, on a effectué des simulations de ce type de fonctionnement auto-piloté en considérant une vitesse de rotation initiale nulle. A titre d'exemple, on a indiqué ci-après les résultats de simulation lorsque l'eau désionisée est utilisée comme couplant. Pour cet exemple, les caractéristiques du moteur sont Ns = 30, Nr = 20, r = 229μm (rayon extérieur du rotor) e = 3 μm (espace entre le rotor et le stator) hr = 45 μm (hauteur des dents rotoriques) hs = 20 μm (hauteur des plots statoriques) l = 10 μm (épaisseur du moteur), hauteur du miroir = 100 μm et inclinaison du miroir = 45°. μm (hauteur des plots statoriques) l = 10 μm (épaisseur du moteur), hauteur du miroir = 100 μm et inclinaison du miroir = 45°.

Simulation du démarrage du micromoteur en mode autopiloté avec une tension d'alimentation de 12.85V

(fonctionnement dans l'eau)

7

# EP 0 806 910 B1

**Revendications**

1. Cathéter d'imagerie échographique endocavitaire comprenant une extrémité proximale (10) située à l'extérieur du patient, une extrémité distale (12) située au voisinage des tissus à explorer et un corps (14) reliant ces deux extrémités (10, 12), l'extrémité distale (12) comprenant une capsule (26) transparente aux ultrasons dans laquelle sont logés un transducteur piezo-électrique fixe (28) et un miroir (30) de déflexion de l'onde ultra sonore, entraîné en rotation par un microactionneur (32) dont le rotor (34) est également logé dans ladite capsule (26), ledit miroir (30) étant déposé directement, sans arbre de transmission du couple à la surface dudit rotor (34) du microactionneur (32) **caractérisé en ce que** l'extrémité distale (12) comprend un fil de guide (24) et **en ce que** le stator (36) dudit microactionneur est logé dans ladite capsule (26).

2. Cathéter selon la revendication 1 **caractérisé en ce que** ledit microactionneur (32) est un moteur électrostatique.

3. Cathéter selon la revendication 2 **caractérisé en ce que** ledit moteur électrostatique est à capacité variable et excitation radiale.

4. Cathéter selon l'une des revendications 2 ou 3 **caractérisé en ce que** le diamètre extérieur dudit moteur est compris entre 0.1 mm et 1 mm, typiquement 0.5 mm et son épaisseur est comprise entre 10 et 100 µm.

5. Cathéter selon la revendication 1 **caractérisé en ce que** ledit microactionneur (32) est un moteur électromagnétique.

6. Cathéter selon l'une quelconque des revendications précédentes **caractérisé en ce que** ledit miroir rotatif (30) est constitué d'une plaque à haute impédence accoustique située au sommet d'un cylindre coupé à environ 45° par rapport à l'axe du transducteur (28) et tournant dans l'axe de ce dernier.

7. Cathéter selon la revendication 6 **caractérisé en ce que** ladite plaque est métallique.

8. Cathéter selon l'une quelconque des revendications 1 à 5 **caractérisé en ce que** ledit miroir (30') est réalisé sous la forme d'un disque, à haute impédence acoustique, posé sur un pied (40) relié directement au rotor (34) dudit microactionneur (32).

9. Cathéter selon les revendications 6 ou 8 **caractérisé en ce que** ledit miroir (30, 30') est constitué d'une couche réfléchissante mince, déposée sur un milieu absorbant les ultrasons.

10. Cathéter selon la revencation 9 **caractérisé en ce que** ladite couche réfléchissante mince est métallique.

11. Cathéter selon l'une quelconque des revendications précédentes **caractérisé en ce que** ledit miroir (30, 30') est de forme plane.

12. Cathéter selon l'une quelconque des revendications précédentes **caractérisé en ce que** ledit miroir (30, 30') est de forme concave.

13. Cathéter selon l'une quelconque des revendications précédentes **caractérisé en ce que** ledit transducteur piezo-électrique (28) est plan ou focalisé.

**Claims**

1. Endocavitary echographic imaging catheter including a proximal end (10) situated outside the patient, a distal end (12) situated in the vicinity of the tissues to be explored and a body (14) linking these two ends (10,12), the distal end (12) including a capsule (26), transparent to ultrasound, in which are housed a fixed piezoelectric transducer (28) and a mirror (30) deflecting the ultrasonic wave, entrained in rotation by a microactuator (32) the rotor (34) of which is also housed in the said capsule (26), the said mirror (30) being placed directly, without a coupling transmission shaft, on the surface of the said rotor (34) of the microactuator (32) **characterized in that** the distal end (12) includes a guide wire (24) and **in that** the stator (36) of the said microactuator is housed in the said capsule (26).

2. Catheter according to claim 1 **characterized in that** the said microactuator (32) is an electrostatic motor.

3. Catheter according to claim 2 **characterized in that** the said electrostatic motor has a variable capacitance and radial excitation.

4. Catheter according to one of claims 2 or 3 **characterized in that** the outside diameter of the said motor is between 0.1 mm and 1 mm, typically 0.5 mm and its thickness is between 10 and 100 μm.

5. Catheter according to claim 1 **characterized in that** the said microactuator (32) is an electromagnetic motor.

6. Catheter according to any one of the previous claims **characterized in that** the said rotating mirror (30) comprises a plate of high acoustic impedance situated at the top of a cylinder cut to approximately 45° relative to the axis of the transducer (28) and rotating in the axis of the latter.

7. Catheter according to claim 6 **characterized in that** the said plate is metallic.

8. Catheter according to any one of claims 1 to 5 **characterized in that** the said mirror (30') is realized in the form of a disk, of high acoustic impedance, placed on a foot (40) connected directly to the rotor (34) of the said microactuator (32).

9. Catheter according to claims 6 or 8 **characterized in that** the said mirror (30, 30') comprises a thin reflecting layer, placed on a medium absorbing ultrasound.

10. Catheter according to claim 9, **characterized in that** the said thin reflecting layer is metallic.

11. Catheter according to any one of the previous claims **characterized in that** the said mirror (30, 30') is planar.

12. Catheter according to any one of the previous claims **characterized in that** the said mirror (30, 30') is concave.

13. Catheter according to any one of the previous claims **characterized in that** the said piezoelectric transducer (28) is plane or focused.


**Patentansprüche**

1. Katheter zur endokavitären Echographie mit einem proximalen Ende (10), das außerhall des Patienten liegt, einem distalen Ende (12), das in der Nähe der zu explorierenden Ge webe liegt, und einem diese zwei Enden (10, 12) verbindenden Körper (14), wobei das distale Ende (12) eine Kapsel (26) aufweist, die für Ultraschallwellen transparent ist und in der ein fester piezo-elektrischer Wandler (28) und ein Spiegel (30) zur Ablenkung der Ultraschallwelle angeordnet sind, wobei der Spiegel durch ein Mikrobetätigungsgerät (32) drehend angetrieben ist, dessen Rotor (34) ebenfalls in der Kapsel (26) untergebracht ist, und der Spiegel (30) direkt ohne Welle zur Übertragung des Drehmoments auf der Oberfläche des Rotors (34) des Mikrobetätigungsgeräts (32) abgelegt ist, **dadurch gekennzeichnet, daß** das distale Ende (12) einen Führungsdraht (24) aufweist und daß der Stator (36) des Mikrobetätigungsgeräts in der Kapsel (26) untergebracht ist.

2. Katheter nach Anspruch 1, **dadurch gekennzeichnet, daß** das Mikrobetätigungsgerät (32) ein elektrostatischer Motor ist.

3. Katheter nach Anspruch 2, **dadurch gekennzeichnet, daß** der elektrostatische Motor eine variable Kapazität und radiale Erregung hat.

4. Katheter nach einem der Ansprüche 2 oder 3, **dadurch gekennzeichnet, daß** der Außendurchmesser des Motors zwischen 0,1 und 1 mm typischerweise 0,5 mm und seine Dicke zwischen 10 und 100 um liegt.

5. Katheter nach Anspruch 1, **dadurch gekennzeichnet, daß** das Mikrobetätigungsgerät (32) ein elektromagnetischer Motor ist.

6. Katheter nach einem der vorangehenden Ansprüche, **dadurch gekennzeichnet, daß** der drehbare Spiegel (30) aus einer Platte mit hoher akustischer Impedanz besteht, die an der Spitze eines mit etwa 45° bezüglich der Achse des Wandlers (28) abgeschnittenen Zylinders angeordnet ist, der sich in der Achse des Wandlers dreht.

**7.** Katheter nach Anspruch 6, **dadurch gekennzeichnet, daß** die Platte aus metallisch ist.

**8.** Katheter nach einem der Ansprüche 1 bis 5, **dadurch gekennzeichnet, daß** der Spiegel (30') in Form einer Scheibe mit hoher akustischer Impedanz hergestellt ist, die auf einem Fuß (40) ruht, der direkt mit dem Rotor (34) des Mikrobetätigungsgeräts (32) verbunden ist.

**9.** Katheter nach den Ansprüchen 6 oder 8, **dadurch gekennzeichnet, daß** der Spiegel (30, 30') aus einer dünnen reflektierenden Schicht besteht, die auf einem ultraschallabsorbierenden Milieu abgeschieden ist.

**10.** Katheter nach Anspruch 9, **dadurch gekennzeichnet, daß** die dünne reflektierende Schicht metallisch ist.

**11.** Katheter nach einem der vorangehenden Ansprüche, **dadurch gekennzeichnet, daß** der Spiegel (30, 30') eine ebene Form hat.

**12.** Katheter nach einem der vorangehenden Ansprüche, **dadurch gekennzeichnet, daß** der Spiegel (30, 30') konkav geformt ist.

**13.** Katheter nach einem der vorangehenden Ansprüche, **dadurch gekennzeichnet, daß** der piezo-elektrische Wandler (28) eben oder fokalisiert ist.

FIG.1

FIG.2

FIG.3

FIG.4

FIG.5

FIG.6

FIG.7

FIG.8